Europäisches Patentamt

European Patent Office

Office européen des brevets

(19)

(11) Veröffentlichungsnummer: **0 339 281**
**A1**

(12) **EUROPÄISCHE PATENTANMELDUNG**

(21) Anmeldenummer: **89105585.7**

(22) Anmeldetag: **30.03.89**

(51) Int. Cl.⁴: **C07C 49/665 , C07C 45/81**

(30) Priorität: **07.04.88 DE 3811635**

(43) Veröffentlichungstag der Anmeldung:
**02.11.89 Patentblatt 89/44**

(84) Benannte Vertragsstaaten:
**CH DE FR GB LI**

(71) Anmelder: **BASF Aktiengesellschaft**
**Carl-Bosch-Strasse 38**
**D-6700 Ludwigshafen(DE)**

(72) Erfinder: **Hoch, Helmut, Dr.**
**Am Wingertsberg 14**
**D-6719 Weisenheim(DE)**
Erfinder: **Kilpper, Gerhard, Dr.**
**Am Tannenhang 1**
**D-6719 Carlsberg(DE)**
Erfinder: **Miederer, Peter, Dr.**
**Barbarossastrasse 3**
**D-6733 Hassloch(DE)**

(54) **Verfahren zur Reinigung von Benzanthron.**

(57) Verfahren zur Reinigung von Benzanthron, bei welchem dem Gemisch aus rohem Benzanthron und einer hochsiedenden organischen Flüssigkeit 1 bis 100 Gew.%, bezogen auf Benzanthron, Wasser, $C_1$- bis $C_5$-Alkanole, zwei- oder mehrwertige Alkohole mit 3 bis 5 C-Atomen, Di- oder Polyethylenglykol, Di- oder Polypropylenglykol, primäre, sekundäre oder tertiäre Amine, Aminoalkohole oder Gemische davon zugegeben werden und das Gemisch auf 100 bis 190 °C erwärmt wird. Der nichtgelöste Anteil wird bei dieser Temperatur abgetrennt und das Benzanthron durch Entfernen der hochsiedenden Flüssigkeit isoliert.

Man erhält Benzanthron mit einem Reingehalt von >97 Gew.% in einer Ausbeute von >97 %, bezogen auf eingesetztes Benzanthron.

EP 0 339 281 A1

EP 0 339 281 A1

## Verfahren zur Reinigung von Benzanthron

Bei der Herstellung von Benzanthron aus Anthrachinon, Glycerin, Eisenpulver und Schwefelsäure wird ein Rohprodukt mit einem Gehalt von ca. 86 Gew.% an Benzanthron erhalten (FIAT, Vol. II, 1313, Seite 31).

Während zur Herstellung von Violanthron rohes Benzanthron ausreichend ist, muß für die anderen Einsatzgebiete, z.B. zur Herstellung der Indanthren Blau-, Grün- und Oliv-Marken ein gereinigtes Benzanthron mit höherem Gehalt an Benzanthron verwendet werden.

Nach FIAT, Vol. II, Nr. 1313, Seite 59 erfolgt die Reinigung von rohem Benzanthron durch Sublimation im Vakuum bei 260 bis 310° C und 2 bis 3 mbar.

Weiterhin ist aus der SU-PS 56 124 ein Verfahren zur Reinigung von Benzanthron durch Umkristallisieren aus organischen Lösungsmitteln, z.B. Chlorbenzol, bekannt. Dabei wird ein Zusatz von Mineralsäure oder Ätzalkali zur Beschleunigung der Filtration empfohlen.

In der SU-PS 1 104 130 wird ein Verfahren zur Herstellung und Reinigung von Benzanthron beschrieben, bei dem das bei der Synthese des Benzanthrons aus Anthrachinon, Glycerin, Schwefelsäure und Reduktionsmittel erhaltene saure Reaktionsgemisch mit Chlorbenzol extrahiert wird. Der Extrakt wird mit Alkali neutralisiert, azeotrop getrocknet, durch Filtration gereinigt und getrocknet.

Weiterhin ist aus der DE-AS 12 45 378 ein Verfahren bekannt, bei dem die zu reinigende Substanz zusammen mit einem hochsiedenden Lösungsmittel bei höherer Temperatur und vermindertem Druck verdampft wird, der Dampf wird kondensiert und das Kondensat in Lösungsmittel und gereinigte organische Substanz getrennt.

Alle bekannten Verfahren haben bei der technischen Durchführung erhebliche Nachteile, so ist z.B. die Vakuumsublimation technisch aufwendig und sehr störanfällig. Um eine hohe Ausbeute an Sublimat, d.h. an gereinigtem Produkt zu erreichen, werden sehr hohe Temperaturen und niedere Drucke benötigt.

Das gleiche gilt für das aus der DE-AS 12 45 378 bekannte Verfahren. Bei diesem ist es zusätzlich erforderlich, aus dem erhaltenen Kondensat - nach dem Beispiel 3 besteht dieses aus Phthalsäuredinonylester und Benzanthron - das Benzanthron abzufiltrieren und durch Waschen mit Methanol von dem Ester zu befreien. Anschließend muß das erhaltene Benzanthron noch getrocknet werden.

Bei dem Verfahren der SU-PS 56 124 wird als Lösungsmittel Chlorbenzol verwendet; Chlorbenzol und ähnliche Verbindungen haben den Nachteil, daß sie sich aus dem Produkt und der Abluft schwer abtrennen lassen und biologisch sehr schwer abbaubar sind. Das gleiche gilt für das Verfahren nach der SU-PS 1 104 130. Außerdem sind in dem Stand der Technik keine Angaben über Ausbeuten und Reinheiten enthalten. Ebenso fehlen Angaben über die erforderlichen Lösungsmittelmengen, insbesondere über das Verhältnis von Benzanthron zu Lösungsmittel, wie auch keine Angaben über die Filtrationseigenschaften des Rückstandes gemacht werden.

Aufgabe der Erfindung war es, ein technisch gut durchführbares Verfahren zur Reinigung von rohem Benzanthron bereitzustellen, das die Nachteile der bekannten Verfahren vermeidet.

Diese Aufgabe wird mit Hilfe des Verfahrens der Erfindung gelöst.

Gegenstand der Erfindung ist ein Verfahren zur Reinigung von Benzanthron durch Erwärmen des rohen Benzanthrons in einer hochsiedenden organischen Flüssigkeit auf Temperaturen von 100 bis 190° C, Abtrennen des bei dieser Temperatur nicht gelösten Anteils und Isolieren des Benzanthrons aus dem Filtrat, das dadurch gekennzeichnet ist, daß man dem Gemisch aus der hochsiedenden organischen Flüssigkeit und dem Rohbenzanthron, bezogen auf Rohbenzanthron, 1 bis 100 Gew.% Wasser, $C_1$- bis $C_5$-Alkanole, zwei- oder mehrwertige Alkohole mit 3 bis 5 C-Atomen, Di- oder Polyethylenglykole, Di- oder Polypropylenglykol, primäre, sekundäre oder tertiäre $C_1$- bis $C_8$-Alkylamine, $C_2$- bis $C_8$-Alkanolamine oder Gemische davon zugibt.

Nach dem Verfahren der Erfindung erhält man Benzanthron mit einem Reingehalt von >97 Gew.% bei einer Ausbeute von >97 %, bezogen auf das im Rohprodukt enthaltene Benzanthron. Ein Vorteil des Verfahrens ist, daß die abgetrennte organische Flüssigkeit direkt wiederverwendet werden kann. Bei Verwendung des bevorzugten Benzoesäuremethylesters kommt hinzu, daß dieser toxikologisch unbedenklich und biologisch gut und praktisch vollständig abbaubar ist.

Das Verfahren hat den weiteren Vorteil, daß durch den Zusatz des Wassers bzw. der anderen Mittel die Filtration beim Abtrennen des Rückstandes um den Faktor 10 bis 20 erhöht, d.h. beschleunigt wird. Dieser Einfluß der genannten Mittel war überraschend und nicht vorherzusehen. Dieser Effekt ist für die Wirtschaftlichkeit des Verfahrens von besonderer Bedeutung.

Im Hinblick auf den Stand der Technik war es überraschend, daß durch ein so einfaches Reinigungsverfahren Benzanthron in praktisch quantitativer Ausbeute mit hohem Reingehalt gewonnen werden kann.

Das Verfahren wird im allgemeinen so ausgeführt, daß man zu der Suspension aus rohem Benzanthron

2

und der organischen Flüssigkeit Wasser, $C_1$- bis $C_5$-Alkanole, zwei- oder mehrwertige Alkohole mit 3 bis 5 C-Atomen, Di- oder Polyethylenglykol, Di- oder Polypropylenglykol, primäre, sekundäre oder tertiäre $C_1$- bis $C_8$-Alkylamino oder Gemische davon in Mengen von 1 bis 100 %, vorzugsweise von 5 bis 50 Gew.%, bezogen auf das trockene rohe Benzanthron, zusetzt, das Gemisch auf 100 bis 190, vorzugsweise 150 bis 160° C erwärmt, wobei ein Teil des Wassers azeotrop mit der organischen Flüssigkeit abdestilliert. Bei dieser Temperatur werden die nicht gelösten Anteile abgetrennt, z.B. durch Filtrieren, und das Filtrat im Vakuum zur Trockene eingeengt.

Das Filtrat wird im Vakuum zur Trockene eingeengt und das feste Benzanthron in bekannter Weise isoliert. Man erhält Benzanthron mit einem Reingehalt von >97 Gew.%. Die Ausbeute beträgt - bezogen auf das im Rohprodukt enthaltene Benzanthron - 97 % und darüber, in der Regel 99 %. Das erhaltene reine Benzanthron enthält nur noch sehr geringe Mengen der organischen Flüssigkeit.

Als organische Flüssigkeiten kommen vorzugsweise Nitrobenzol, Tributylamin und Benzoesäuremethylester in Betracht. Benzoesäuremethylester ist besonders geeignet und daher besonders bevorzugt. Die in der technischen Qualität enthaltenen Verunreinigungen wie Benzaldehyd, p-Tolylaldehyd, Tolylester, Essigsäure u.a. Verbindungen stören nicht.

Als Zusätze kommen neben Wasser $C_1$- bis $C_5$-Alkanole, zwei- oder mehrwertige Alkohole mit 3 bis 5. C-Atomen, Di- oder Polyethylenglkyol, Di- oder Polypropylenglykol, primäre, sekundäre oder tertiäre $C_1$- bis $C_8$-Alkylamine, $C_2$- bis $C_8$-Alkanolamine oder Gemische dieser Mittel in Betracht.

Im einzelnen sind z.B. zu nennen:

$C_1$- bis $C_5$-Alkanole wie Methanol, Ethanol, n- und iso-Propanol, n- und i-Butanol, n- und i-Pentanol;

Glykole wie Ethylenglykol, Di-, Tri- und Tetraethylenglykol, Polyethylenglykole, 1,2-Propylenglykol, Di-, Tri- und Tetrapropylenglykol-1,2;

zwei- und mehrwertige Alkohole mit 3 bis 5 C-Atomen, wie Glycerin, Propandiol-1,3, Butandiol-1,2, -1,3 und -1,4, die Pentandiole-1,2, -1,3 und -1,5;

$C_1$- bis $C_8$-Alkylamine und $C_2$- bis $C_8$-Alkanolamine wie Dimethyl-, Trimethyl-, Diethyl-, Triethyl-, Propyl-, Di-n-propyl-, Di-i-propyl, Tri-n-propyl-, Tri-i-propyl-, Butyl-, Di-n-butyl-, Di-i-butyl-, Tri-n-butyl-, Tri-i-butyl-, Pentyl-, Di-n-pentyl-, Di-i-pentyl, Tri-n-pentyl-, Tri-i-pentyl, Hexyl-, Di-n-hexyl-, Di-i-hexyl-, Tri-n-hexyl-, Tri-i-hexyl-, Heptyl-, Di-n-heptyl-, Di-i-heptyl-, Tri-n-heptyl-, Tri-i-heptyl-, Octyl-, Di-n-octyl-, Di-i-octyl, Tri-n-octyl-, Tri-i-octyl-, 2-Ethylhexyl-, Di-2-ethylylhexyl-, Cyclohexyl, Di-cyclohexyl-, Tri-cyclohexyl-, N-Methyl-N-cyclohexyl- und N-Ethyl-N-cyclohexylamine sowie Ethanolamin, Diethanolamin, Triethanolamin, i-Propanolamin, Di-i-propanolamin, Tri-i-propanolamin, N-Methylethanolamin, N,N-Dimethylethanolamin und N-Methyldiethanolamin.

Als Zusätze sind aufgrund der besonders guten Wirkung Wasser und Ethylenglykol bevorzugt.

In einer besonders bevorzugten Verfahrensvariante erfolgt die Reinigung in Benzoesäuremethylester unter Zusatz von Wasser oder Ethylenglykol.

Ein weiterer Vorteil des Verfahrens gemäß der Erfindung liegt darin, daß das bei der Benzanthron-Synthese erhaltene feuchte rohe Benzanthron direkt zur Reinigung verwendet werden kann. Dabei wird die Trocknung und die Reinigung in einem Verfahrensschritt durchgeführt.

Die letztgenannte Verfahrensvariante wird in der Regel so ausgeführt, daß man das rohe feuchte Benzanthron bei Raumtemperatur in der 3- bis 4-fachen Gewichtsmenge der organischen Flüssigkeit, vorzugsweise Benzoesäuremethylester - bezogen auf Benzanthron roh gerechnet trocken - suspendiert und dann das Gemisch unter Abdestillieren von Wasser auf eine Temperatur von 160° C aufheizt. Bei 160° C wird dann der Rückstand abfiltriert. Bei dieser Variante fällt der Rückstand bei Verwendung von Benzoesäuremethylester als organische Flüssigkeit in einer groben, hervorragend filtrierbaren Form an. Das Filtrat wird im Vakuum zur Trockene eingeengt und das Benzanthron in bekannter Weise isoliert. Der Reingehalt des Benzanthrons beträgt 97 bis 99 %, die Ausbeute liegt - -bezogen auf das im Rohprodukt enthaltene - Benzanthron bei 99 % und darüber.

Der besondere Vorteil des Verfahrens der Erfindung besteht darin, daß die organische Flüssigkeit, insbesondere der Benzoesäuremethylester ohne weitere Reinigung wiederverwendet werden kann. Die Verluste sind sehr gering.

Benzoesäuremethylester ist biologisch sehr gut abbaubar (Eliminierbarkeit >98 %) und toxikologisch unbedenklich. Da Benzoesäuremethylester durch verdünnte Alkalilaugen rasch verseift wird, kann die Abluft problemlos durch nachgeschaltete Wäscher mit verdünnter Natronlauge gereinigt werden.

Das Verfahren soll durch die folgenden Beispiele zusätzlich erläutert werden. Die angegebenen Teile sind Gewichtsteile und die Prozente beziehen sich auf das Gewicht.

Beispiel 1

In 1 200 Teile Benzoesäuremethylester (Gehalt 99,5 %) werden bei Raumtemperatur 341 Teile Benzanthron roh (Gehalt: 88 %); entsprechend 300 Teile Benzanthron roh ber. 100 %) eingetragen. Nach dem Zugeben von 15 Teilen Wasser wird auf 160°C geheizt. Nach 1 Stunde bei dieser Temperatur wird bei 160°C das Ungelöste abfiltriert. Filtrationsdauer: 10 Sekunden. Das Filtrat wird bei 25 bis 30 mbar und 120 bis 140°C zur Trockene eingedampft. Man erhält 304 Teile Benzanthron mit einem Gehalt von 98 % (bestimmt durch HPLC) entsprechend 298 Teile ber. 100 %. Die Ausbeute beträgt 99,3 %, bezogen auf eingesetztes Benzanthron. Das Benzanthron enthält weniger als 1 % Benzoesäuremethylester.

Arbeitet man ohne Wasser, werden zum Absaugen des Ungelösten 4,5 Minuten benötigt.

Beispiel 2

Man verfährt wie in Beispiel 1, verwendet jedoch die gleiche Menge technischen Benzoesäuremethylester mit einem Gehalt von 92 % und erhält Benzanthron in gleicher Ausbeute und Qualität wie bei Beispiel 1.

Beispiel 3

In 1 200 Teile technischem Benzoesäuremethylester (Gehalt 92 %) werden bei Raumtemperatur 568 Teile Benzanthron roh feucht (Trockengehalt 60 %, Reingehalt 88 %, bezogen auf Trockensubstanz) entsprechend 300 Teile Benzanthron ber. 100 % eingetragen. Unter Durchleiten eines schwachen Stickstoffstromes wird auf 120°C aufgeheizt, wobei Wasser azeotrop mit geringen Mengen Benzoesäuremethylester abdestilliert. Anschließend wird die Temperatur auf 160°C erhöht und nach 1 Stunde bei 160°C das Ungelöste abfiltriert. Die Filtrationszeit beträgt 5 Sekunden. Das Filtrat wird wie in Beispiel 1 zur Trockene eingeengt. Ausbeute: 305 Teile Benzanthron mit einem Gehalt von 97,5 % (nach HPLC) $\hat{=}$ 297 Teile Benzanthron ber. 100 % (99,1 % v. Einsatz).

Die Reinigung wurde mehrere Male wiederholt, dabei lag die Dauer der Filtration zwischen 5 und 10 Sekunden.

Beispiel 4

Es wurde wie in Beispiel 1 verfahren, jedoch wurden vor dem Aufheizen anstelle der 15 Teile Wasser jeweils 5 Teile der in der folgenden Tabelle aufgeführten Stoffe zugegeben.

Ausbeuten und Reinheit an Benzanthron entsprechend dem nach Beispiel 3 erhaltenen Produkt.

| Zusatzstoff | Dauer der Filtration |
|---|---|
| Ethylenglykol | 8 Sek. |
| Triethylamin | 12 Sek. |
| Diethanolamin | 13 Sek. |
| Triethanolamin | 8 Sek. |

Entsprechende Ergebnisse erhält man, wenn man statt des reinen Benzoesäuremethylesters den technischen Ester mit einem Gehalt von 92 % verwendet.

Beispiel 5

Es wurde wie in Beispiel 1 verfahren, jedoch wurde der Benzoesäuremethylester durch die gleiche Menge Nitrobenzol ersetzt. Vor dem Aufheizen wurden jeweils 15 Teile der in der folgenden Tabelle genannten Zusatzstoffe zugegeben.

Die Ausbeute an Benzanthron beträgt 99 %, der Reingehalt 97,5 % (HPLC) Produkt.

| Zusatzstoff | Dauer der Filtration |
|---|---|
| Wasser | 15 Sek. |
| Ethylenglykol | 12 Sek. |
| Triethylamin | 15 Sek. |
| Diethanolamin | 12 Sek. |
| Triethanolamin | 10 Sek. |

Ohne Verwendung der Mittel beträgt die Dauer der Filtration 5 Minuten.

Beispiel 6

Es wurde wie in Beispiel 1 verfahren, jedoch wurden anstelle von Benzoesäuremethylester 2400 Teile Tributylamin verwendet. Die Filtrationsdauer beträgt 10 Sek. Die Ausbeute an Benzanthron beträgt 98 %, der Reinheitsgehalt 98,5 %.

**Ansprüche**

1. Verfahren zur Reinigung von Benzanthron durch Erwärmen des rohen Benzanthrons in einer hochsiedenden organischen Flüssigkeit auf Temperaturen von 100 bis 190°C, Abtrennen des bei dieser Temperatur nicht gelösten Anteils und Isolieren des Benzanthrons aus dem Filtrat, dadurch gekennzeichnet, daß man dem Gemisch aus der hochsiedenden organischen Flüssigkeit und dem Rohbenzanthron, bezogen auf Rohbenzanthron, 1 bis 100 Gew.% Wasser, $C_1$- bis $C_5$-Alkanole, zwei- oder mehrwertige Alkohole mit 3 bis 5 C-Atomen, Di- oder Polyethylenglykol, Di- oder Polypropylenglykol, primäre, sekundäre oder tertiäre $C_1$- bis $C_8$-Alkylamine, $C_2$- bis $C_8$-Alkanolamine oder Gemische davon zugibt.

2. Verfahren gemäß Anspruch 1, dadurch gekennzeichnet, daß man als hochsiedende organische Flüssigkeit Nitrobenzol, Tributylamin und/oder Benzoesäuremethylester verwendet.

3. Verfahren gemäß Anspruch 1, dadurch gekennzeichnet, daß man als organische Flüssigkeit Benzoesäuremethylester verwendet.

4. Verfahren gemäß Anspruch 1, 2 oder 3, dadurch gekennzeichnet, daß man zu dem Gemisch aus der organischen Flüssigkeit und dem Rohbenzanthron Wasser oder Ethylenglykol gibt.

5. Verfahren gemäß Anspruch 1, 2, 3 oder 4, dadurch gekennzeichnet, daß man, bezogen auf rohes Benzanthron, die 2- bis 10-fache Gewichtsmenge an organischer Flüssigkeit verwendet.

6. Verfahren gemäß Anspruch 3, dadurch gekennzeichnet, daß man, bezogen auf rohes Benzanthron, die 3- bis 4-fache Gewichtsmenge an Benzoesäuremethylester verwendet.

7. Verfahren gemäß Anspruch 6, dadurch gekennzeichnet, daß man dem Gemisch aus Benzoesäuremethylester und rohem Benzanthron Wasser zusetzt und dann das Gemisch auf 150 bis 160°C erwärmt.

8. Verfahren gemäß den Ansprüchen 1 bis 7, dadurch gekennzeichnet, daß man rohes, wasserfeuchtes Benzanthron verwendet und das Gemisch mit der organischen Flüssigkeit unter Abdestillieren von Wasser auf 150 bis 160°C erwärmt und bei dieser Temperatur den nicht gelösten Anteil abtrennt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | CHEMICAL ABSTRACTS Band 101, Nr. 21, 19. November 1984, Zusammenfassung Nr. 191407z, Columbus, Ohio, USA; & SU -A - 1104130, 23.07.1984 --- | 1 | C 07 C 49/665 C 07 C 45/81 |
| A | CHEMICAL ABSTRACTS Band 99, Nr. 26, 26. Dezember 1983, Zusammenfassung Nr. 214163y, Columbus, Ohio, USA; & CS - A - 212039, 15.04.1983 --- | 1 | |
| D,A | DE-B-1 245 378 (BASF) * Spalten 4,5; Beispiele 3,4,12 * --- | 1 | |
| A | US-A-1 365 024 (DANIELS) * Ansprüche * --- | 1 | |
| A | US-A-1 900 972 (BECKER) * Ansprüche * --- | 1 | RECHERCHIERTE SACHGEBIETE (Int. Cl.4) |
| A | FR-A- 668 085 (NEWPORT CO.) * Anspruch * ----- | 1 | C 07 C 49/00 C 07 C 45/00 |

Der vorliegende Recherchenbericht wurde für alle Patentansprüche erstellt

| Recherchenort | Abschlußdatum der Recherche | Prüfer |
|---|---|---|
| BERLIN | 14-08-1989 | PROBERT C.L. |